# EUROPEAN PATENT APPLICATION

(11) **EP 1 923 039 A1**
(43) Date of publication of application: **21.05.2008**
(21) Application number: 06023108.1
(22) Date of filing: 07.11.2006
(51) Int. Cl.: A61K 8/04, A61Q 5/06, A61Q 5/12

(54) **Conditioning mousse**

(71) Applicant: KPSS-Kao Professional Salon Services GmbH, 64297 Darmstadt (DE)
(72) Inventor: Hoffmann, Martin, 64673 Zwingenbreg (DE); Ast, Andrea, 64283 Darmstadt (DE)

(57) **Abstract**

The present invention is related to composition for conditioning keratin fibres especially human hair with reduced bulk density. The inventors of the present invention have found out that a conditioning composition comprising at least one shear thinning polymer and at least one conditioning compound and having reduced bulk density solves the above mentioned problems in an outstanding environmental compatible way. Since the reduced bulk density the composition is perceived light which for example cause no weigh down effect. The reduced bulk density at the same time improves and optimizes spreadability of the composition to the applied sites and therewith optimizes as well the uptake, in other words, the effect of such composition. Accordingly the subject of the present invention is an aqueous conditioning composition for keratin fibres especially human hair comprising at least one shear thinning polymer, at least one conditioning compound, at least one gas and having a bulk density in the range of 0.3 to 0.9 g/ml measured at 20°C wherein composition is not released or stored under pressure.

## Description

The present invention is related to composition for conditioning keratin fibres especially human hair with reduced bulk density.

Conditioning compositions for hair have been known in the art and have been widely used. They are used to repair damaged hair and/or to improve certain properties of hair such as combability (primarily), shine, volume, body, elasticity etc. The results have however been found variable depending on physical status of hair i.e. thick or thin, damaged or healthy. Since the rich content and deposition of the active matters is in quite large quantities some of the consumers have found that although combing becomes easier but hair often feels heavy i.e. looses volume and body, does not feel natural upon touching.

Since the above reasons, consumers having especially fine hair have been remaining using such conditioning compositions. The present invention aims proving a solution to the above problems.

The present inventors have surprisingly found out that the perception of conditioning composition is very much dependent on the appearance and physical status of such compositions. In cases where a thick heavy masses are offered to the consumers especially having fine hair, they refrain from using such compositions because of the above mentioned disadvantageous. On the other hand they do use conditioning compositions delivered for example in form of foam with comparably identical components.

Foam type of conditioners require that the composition is confectioned in form of an aerosol and require therefore certain storage and production facilities which increases costs enormously.

The inventors of the present invention have found out that a conditioning composition comprising at least one shear thickening polymer and at least one conditioning compound and having reduced bulk density solves the above mentioned problems in an outstanding environmental compatible way. Since the reduced bulk density the composition is perceived light which for example cause no weigh down effect. The reduced bulk density at the same time improves and optimizes spreadability of the composition to the applied sites and therewith optimizes as well the uptake, in other words, the effect of such composition.

Accordingly the subject of the present invention is an aqueous conditioning composition for keratin fibres especially human hair comprising at least one shear thinning polymer, at least one conditioning compound, at least one gas and having a bulk density in the range of 0.3 to 0.9 g/ml measured at 20°C wherein composition is not released or stored under pressure.

Further subject of the present invention is process for conditioning hair wherein a composition comprising at least one shear thing polymer, at least one conditioning compound, at least one gas and having a bulk density in the range of 0.3 to 0.9 g/ml and not released from a contained under pressure is applied onto previously wetted or cleansed using a cleansing composition hair and after a processing time of 30 seconds to 30 min at a temperature range of 20 to 45°C, rinsed off from hair.

Still further subject of the present invention is a ready to use product comprising at least one shear thinning polymer, at least one conditioning compound, at least one gas and having a bulk density in the range of 0.3 to 0.9 g/ml and a vessel having appropriate volume and a cap which enables storing the composition stably under atmospheric pressure.

Another subject of the present invention is process for producing a conditioning composition for keratin fibres especially human hair wherein a composition comprising at least one shear thinning polymer and at least one conditioning compound is mixed with an appropriate amount of gas to result in a bulk density in the range of 0.3 to 0.9 g/ml.

Still another subject of the present invention is the use of composition comprising at least one shear thinning polymer, at least one conditioning compound, at least one gas and having a bulk density in the range of 0.3 to 0.9 g/ml wherein composition is not released or stored under pressure, for conditioning keratin fibres especially human hair.

Within the meaning of the present invention with the term bulk density it is meant the amount of composition per unit volume of the conditioner. The unit is g/ml or equally g/cm3. All bulk density values given are measured at 20°C unless otherwise indicated.

Bulk density of conditioner compositions according to the present invention is in the range of 0.3 to 0.9 g/ml, preferably 0.4 to 0.75 g/ml and more preferably 0.45 to 0.7 g/ml and most preferably 0.5 to 0.65 g/ml.

In order to reach the above given bulk density values a gas is included into the composition by mixing, dispersing the gas under positive pressure. Suitable are air, nitrogen, carbon dioxide. Preferred are air and nitrogen. Most preferred is nitrogen.

When selecting gas to be dispersed in the compositions of the present invention attention should be paid to the stability and especially chemical stability of compounds in the compositions. For example, when oxidation sensitive compounds are included such as unsaturated fatty acids air should not be used because it may cause oxidation of unsaturated fatty acids. The example given should not bring any limitation. Skilled worker know very well or able to define oxidation sensitive ingredients.

The size of the dispersed gas is not a parameter but should preferably be in the range of a 100µ to 5 mm, preferably 250 µ to 3 mm.

The compositions of the present invention comprise a shear thing polymer. In principal all shear thinning polymers are suitable either natural or synthetic ones. Nonlimiting examples to the suitable shear thinning polymers are acrylate polymers sold under the trade name Carbopol which are anionic polymers, polyquaternium 37 sold under the trade name Ultragel 300, another cationic acrylate polymer sold under the trade name Carbopol Aqua CC. Preferred are cationic shear thinning polymers. Example to the natural shear thinning polymer is xanthan gum. Compositions can also comprise mixture of the shear thinning polymers. Attention should be paid compatibility of the selected shear thinning polymer with the rest of the composition.

Concentration of the shear thinning polymer is in the range of 0.05 to 5% by weight, preferably 0.1 to 3% by weight and more preferably 0.25 to 2.5% and most preferably 0.25 to 2% by weight calculated to the total composition.

The composition of the present invention comprises at least one hair-conditioning agents. Conditioning agents can be selected from oily substances, non-ionic substances, cationic amphiphilic ingredients, cationic polymers or their mixtures.

Oily substances are selected from such as silicone oils, either volatile or non-volatile, natural and synthetic oils. Among silicone oils those can be added to the compositions include either volatile or non-volatile such as dimethicone, dimethiconol, polydimethylsiloxane, DC fluid ranges from Dow Corning, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₅ CO (O CH₂ CH₂)ₙ OH

or

R₅ CO (O CH₂ CH₂)n O OC R₆

where R₅ and R₆ are independent from each other saturated, unsaturated or branched or non-branched alkyl chain with 7 to 21 C atoms and n is typically 2 - 100.

Conditioning agents can also be cationic polymers. Suitable cationic polymers are those of best known with their INCI category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquaternium 6 and polyquaternium 7.

The cationic polymers also include the quaternized products of graft polymers from organopolysiloxanes and polyethyl oxazolines described in EP-A 524 612 and EP-A 640 643.

Another suitable cationic polymers are those silicone polymers containing at least one primary, secondary, tertiary and/or quaternary amino and/or ammonium group. Examples are Polysilicone-9, and amodimethicone.

Conditioning composition of the present invention can comprise one or more cationic surfactant(s) as conditioner presented with the general formula 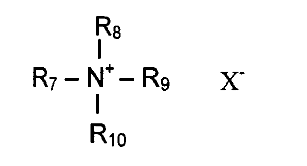 where R₇ is a saturated or unsaturated, branched or non-branched alkyl chain with 8-22 C atoms or

R₁₁ CO NH (CH₂)ₙ

where R₁₁ is saturated or unsaturated, branched or non-branched alkyl chain with 7-21 C atoms and n has value of 1 - 4,
or

R₁₂ CO O (CH₂)ₙ

where R₁₂ is saturated or unsaturated, branched or non-branched alkyl chain with 7-21 C atoms and n has value of 1 - 4, and
R₈ is hydrogen or unsaturated or saturated, branched or non-branched alkyl chain with 1 - 4 C atoms or

R₁₁ CO NH (CH₂)ₙ

or

R₁₂ CO O (CH₂)ₙ

where R₁₁, R₁₂ and n are same as above.

R₉ and R₁₀ are hydrogen or lower alkyl chain with 1 to 4 carbon atoms, or ethoxy or propoxy groups with 1 to 4 repeating units and X is anion such as chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.

Further suitable cationizable conditioning compounds are those with one or more long chain (C12 to c22) alkyl or alkoyl or alkyl amido alky chain amines which are protonated and therefore cationic at acidic pHs.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Further suitable conditioning compounds according to the present invention are those known with general name lecithin obtained either synthetic or natural resources such as soja beans or egg yolk. Examples to the individual ones are phosphatidylcholine, phosphatidyl glycerol, phosphatidyl serine, and phosphatidyl inositol.

Typical concentration range for any of those conditioners of cationic polymers, silicon oil and derivatives and cationic surfactants can be 0.01 - 5% by weight, preferably 0.01 - 3.5% by weight, more preferably 0.05 - 2.5% and most preferably 0.1 - 1.5% by weight calculated to the total composition.

The compositions of the present invention can be transparent or non-transparent. -Transparency is judged macroscopically with naked eye in a vessel with 3cm thickness. Transparency may certainly be judged using spectroscopic methods, however, presence of dispersed gas should be taken into account as especially the small gas bubbles may scatter light and therefore cause unrealistic results.

As a non-transparent composition creams, emulsions are the most preferred ones. In case of emulsions (synonymous cream) compositions comprise further preferably at least one emulsifier. Suitable emulsifiers are cationic, non-ionic, amphoteric surfactants or their mixtures. Anionic surfactants are also suitable as emulsifiers but they are less preferred. The most preferred emulsifiers are cationic and non-ionic surfactants or their mixtures. In addition crude lecithin either from natural resources such as sja beans or egg yolk or more particularly phosphatidyl choline is also preferred emulsifier in addition to their conditioning effects.

Cationic surfactants are mentioned above. Among those, suitable emulsifiers are those of single long alkyl chain containing ones such as cetyltrimethylammonium chloride.

Suitable non-ionic surfactants are alkyl polyglucosides of the general formula

R₇ - O - (R₈O)ₙ O - Zₓ

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Among the non-ionic surfactants mentioned above fatty alcohol ethoxylates are the most preferred ones. Above mentioned non-ionic surfactants can also be used as a mixture of one category such as several ethoxylated fatty alcohols or several categories such as mixture of alkyl polyglucoside and ethoxylated fatty alcohol.

As further surfactant component as emulsifier, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate.

Further emulsifiers suitable, although less preferred, within the meaning of the present invention are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful in the liquid cleansing compositions are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₉ - (C₂H₄O)ₙ - O - CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted.

Further anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Most preferred emulsifiers are cationic and non-ionic surfactants.

Concentration of emulsifier in the compositions according to present invention is in the range of 0.05 to 10%, preferably 0.1 to 7.5% and more preferably 0.25 to 5% by weight calculated to total composition.

Emulsions can contain one or more fatty alcohols. Another preferred form is oil in water emulsion. Emulsions according to the present invention preferably comprise at least one fatty alcohol with linear of branched alkyl chain. Suitable ones are fatty alcohols having 12 to 22 C atoms in its alkyl chain. Examples are myristyl alcohol, cetyl alcohol, stearyl alcohol, behenyl alcohol and their mixtures. Preferred are cetyl, stearyl and behenyl alcohol and their mixtures i.e. cetearyl alcohol. Fatty alcohols may be included into the compositions of the present invention at a concentration of 0.1 to 20%, preferably 0.5 to 15% and more preferably 1 to 10% by weight calculated to total composition.

The compositions according to the present invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin".

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapone" products and "Herbasol^{R}". Extracts and the preparation thereof are also described in "Hagers Handbuch der pharmazeutischen Praxis", 4th Ed.

The compositions may contain organic solvents such as ethanol. propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the composition should not exceed 5% by weight. It should be noted that penetration enhancers are useful for both cleansing and after shampoo conditioning preparations. It is obvious that the concentration in the cleansing compositions is usually lower than in the conditioning preparations.

Compositions of the present invention can comprise UV filters either for stabilization of the product colour or for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzophenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzylidenebornane-2-one and the salts thereof and/or 3-(4'-methyl benzylidene)-DL-campher, polysilicone-15. The preferred amount of the UV-absorber ranges from about 0.01 % to 2.5%, more preferably from 0.05 % to 1 % by weight, calculated to the total composition.

The compositions of the present invention can comprise hair-restructuring agents. The hair restructuring agents preferred are especially the ones disclosed in the German patent DE 197 51 550 C2. Namely they are ceramide type of compounds, fatty acids and phytosterol or their mixtures.

Preferred ceramide compound is cetyl-PG-hydroxyethylpalmitamide.

Preferred fatty acids are those with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.01 to 1% by weight calculated to the total weight of the composition. The fatty acids may be contained at a level of 0.01 to 2.5% and especially 0.01 to 1% by weight calculated to the total weight of the composition. Phytosterol concentration of the conditioners is less than 1 % and preferably in the range of 0.01 to 0.5% by weight calculated to the total weight of the composition. It should be noted without limiting the use of those ingredients the effect of those hair restructuring ingredients is especially elevated when used in combination with penetration enhancers.

The pH of the compositions according to the invention is in the range of 2 to 7, preferably 3 to 6, more preferably 3 to 5. For adjusting the pH of the said compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. Typically concentration for acids can be 0.01 - 3% by weight, preferably 0.05 - 2% by weight, more preferably 0.05 - 1.5% by weight calculated to the total composition. The pH of the composition can also be adjusted to the required pH by using alkaline solution such as sodium hydroxide, ammonium hydroxide, potassium hydroxide or their salts with those acids mentioned above in the case that at the selected acid concentration pH of the composition is lower than that of the aimed value.

Compositions of the present invention can comprise particles of synthetic mica coated with metal oxide or oxides and having a volume particle size distribution in the range of 1 to 750 µm at a concentration of 0.01 to 10% by weight, calculated to total composition.

Suitable metal oxide or oxides for coating synthetic mica are titanium dioxide, chromium oxide, ferric oxide or mixtures thereof. In the present invention the preferred is synthetic mica coated with titanium dioxide. Such materials are commercially available from Sun Chemical Corporation and known with their INCI names Synthetic Fluorphologopite.

The volume particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

In a further preferred form of the present invention the compositions comprise at least one direct dye for colouring hair. Suitable direct dyes are cationic, anionic, neutral dyes and their mixtures as available commercially from various suppliers and used mainly in semipermanent hair coloration.

Cationic dyes are the most preferred ones for the purpose of dyeing hair. Nonlimiting examples are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Further suitable direct dyes are anionic dyes especially at pH values in the range of 2 to 3.5. Suitable nonlimiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No. 15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used as hair colorant within the meaning of the present invention for example henna (red or black), alkanna root, laccaic acid, indigo, logwood powder, madder root and rhubarb powder, etc.

It should be noted that the above dyestuffs are also suitable for use in mixture. When using direct dyes of various categories, their compatibility must be checked.

Concentration of direct dyes in the compositions of the present invention is within the range of 0.001 to 5%, preferably 0.01 to 3% and more preferably 0.05 to 2%, and most preferably 0.1 to 1% by weight calculated to total composition.

It should be noted that anionic, cationic, neutral and plant (natural) dyes are as well used in combination with each other.

The concentration of total dyestuffs, one or more and in mixture with any other either anionic, or cationic, or neutral or natural, is lower than 0.25%, preferably lower than 0.2% and more preferably lower than 0.15% and most preferably lower than 0.1% by weight calculated to the total composition. As the subject matter of the,present invention is colour stabilization, the composition in any case comprise one or more dyestuffs and the concentration can be as low as 0.00001 % by weight calculated to total composition.

Among all the dyestuffs disclosed above, anionic dyes are found to be the most suitable and preferred ones.

Furthermore compositions of the present invention can comprise all substances customarily found in such preparations. Examples of such substances are complexing agents, dyestuffs preferably non-substantive for colouring composition, preservatives, fragrances, etc.

Compositions of the present invention can be used as either rinse off or leave in composition. The preferred usage is rinse off and more preferably after cleansing hair. On the other hand usage without previous cleansing should not be excluded. Accordingly, process for conditioning keratin fibres especially human hair, consists of washing hair, preferably with a cleansing composition and then applying a composition comprising at least one shear thinning polymer, at least one conditioning compound, at least one gas and having a bulk density in the range of 0.3 to 0.9 g/ml
wherein composition is not released or stored under pressure, after processing for 30 sec to 30 min, preferably 1 min to 15 min and more preferably 3 minute to 10 min at ambient temperature or at a temperature in the range of 20 to 45°C, rinsed off.

Following examples are to illustrate the invention but not to limit.

### Example 1

| | % by weight |
|---|---|
| Polyquaternium-37 | 2.0 |
| Behentrimonium chloride | 1.0 |
| Citric acid/Sodium hydroxide | q.s to pH 4.0 |
| Fragrance preservative | q.s. |
| Water | q.s to 100 |

The above composition was prepared by dissolving polyquaternium-37 and behentrimonium chloride separately in portions of water and combining together after adding fragrance into behentrimonium chloride solution/dispersion. Finally remaining water and preservative was added and pH was adjusted. The composition produced was further mixed under nitrogen atmosphere at a positive pressure of 1 bar. The obtained product had a bulk density of 0.615 g/ml.

A portion of the product was taken as a comparative sample before mixing the nitrogen gas. The bulk density was 1.005 g/ml.

Example 1 and Comparative example was compared in a half side test with 10 female volunteers with shoulder length hair. Firstly the hair of the volunteers was washed with a commercially available shampoo and subsequently conditioner compositions were applied to each half side. The amount was around 5 g each half side. After processing of 5 min at ambient temperature the products were rinsed off and 2 hair dressers were asked to give their opinion on hair conditioning in towel dry and dried state. Hair dressers first comment was that example 1 was easy to apply and spread onto hair. It was also clear that example 1 was taken up by hair judged on the basis of appearance on the hair, i.e. how much product was visible - felt by toughing. Furthermore the side with example 1 was found to be smoother and have better elasticity, volume and shine. Combability was generally found to be comparable for both sides with slight tendency that example 1 applied side was slightly better combable. Positive comments from hair dressers were on appearance and consistency of the conditioner composition when applying by hands. It was mentioned that light conditioning benefits were visualised with appearance and foam-cream like feel in hands.

### Example 2

| | % by weight |
|---|---|
| Ceterayl alcohol | 5.0 |
| Polyquaternium-37 | 2.0 |
| Behentrimonium chloride | 1.0 |
| Dimethicone | 0.2 |
| Lecithin | 0.1 |
| Panthenol | 0.5 |
| Lactic acid/Sodium hydroxide | q.s to pH 3.5 |
| Fragrance preservative | q.s. |
| Water | q.s to 100 |

The above composition was prepared by dissolving polyquaternium-37 in part of water. In another vessel cetearyl alcohol, lecithin, dimethicone and behentrimonium chloride were emulsified and polyquaternium 37 solution/dispersion was added. Finally panthenol, fragrance, remaining water and preservative was added and pH was adjusted. The composition produced was further mixed under nitrogen atmosphere at a positive pressure of 1 bar. The obtained product had a bulk density of 0.572 g/ml.

In half side comparative test as in example 1 similar results were found out.

### Example 3

| | % by weight |
|---|---|
| Ceterayl alcohol | 5.0 |
| Polyquaternium-37 | 2.0 |
| Ceteareth 20 | 2.0 |
| Stearamidopropyldimethlyamine | 2.0 |
| Amodimethicone | 0.5 |
| Lecithin | 0.1 |
| Panthenol | 0.5 |
| Benzophenone-3 | 0.2 |
| Lactic acid/Sodium hydroxide | q.s to pH 3.5 |
| Fragrance preservative | q.s. |
| Water | q.s to 100 |

The above composition produced in a similar was as in example 2 and had a bulk density of 0.52.

### Example 4

| | % by weight |
|---|---|
| Ceterayl alcohol | 6.0 |
| Polyquaternium-37 | 2.0 |
| Ceteareth 20 | 2.0 |
| Cetrimonium chloride | 1.0 |
| hydroxyethylmonium methosulfate | 2.0 |
| Amodimethicone | 0.5 |
| Lecithin | 0.1 |
| Glycerine | 0.5 |
| Octylmethoxycinnamate | 0.3 |
| Lactic acid/Sodium hydroxide | q.s to pH 3.8 |
| Fragrance preservative | q.s. |
| Water | q.s to 100 |

The above composition produced in a similar was as in example 2 and had a bulk density of 0.54.

Above composition was filled in a jar and its stability was followed at 5, room temperature and 40°C for 3 months. At all three temperatures tested it was found to be stable within the test period as no major changes were observed in appearance, pH, and bulk density. At the end of the 3 month period the bulk density (measured after equilibration at 20°C for a day) of sample stored at 5°C was unchanged, room temperature was 0.605, and at 40°C 0.621 g/ml. Room temperature during the test period was approximately 20°C.

### Example 5

| | % by weight |
|---|---|
| Ceterayl alcohol | 6.0 |
| Polyquaternium-37 | 2.0 |
| Ceteareth 20 | 2.0 |
| Cetrimonium chloride | 1.0 |
| hydroxyethylmonium methosulfate | 2.0 |
| Amodimethicone | 0.5 |
| Lecithin | 0.1 |
| Glycerine | 0.5 |
| Octylmethoxycinnamate | 0.3 |
| Basic red 51 | 0.1 |
| Lactic acid/Sodium hydroxide | q.s to pH 3.8 |
| Fragrance preservative | q.s. |
| Water | q.s to 100 |

The above composition produced in a similar was as in example 2 and had a bulk density of 0.58.

The composition gave medium blond hair intensive red touch.

### Example 5

| | % by weight |
|---|---|
| Ceterayl alcohol | 6.0 |
| Polyquaternium-37 | 2.0 |
| Ceteareth 20 | 2.0 |
| Cetrimonium chloride | 1.0 |
| hydroxyethylmonium methosulfate | 2.0 |
| Amodimethicone | 0.5 |
| Lecithin | 0.1 |
| Glycerine | 0.5 |
| Octylmethoxycinnamate | 0.3 |
| Basic yellow 87 | 0.05 |
| Basic orange 31 | 0.02 |
| Lactic acid/Sodium hydroxide | q.s to pH 3.8 |
| Fragrance preservative | q.s. |
| Water | q.s to 100 |

The above composition produced in a similar was as in example 2 and had a bulk density of 0.56.

The composition gave light blond hair intensive blond reflex.

## Claims

1. Aqueous conditioning composition for keratin fibres especially human hair **characterised in that** it comprises at least one shear thinning polymer, at least one conditioning compound, at least one gas and having a bulk density in the range of 0.3 to 0.9 g/ml measured at 20°C wherein composition is not released or stored under pressure.

2. Composition according to claim 1 **characterised in that** shear thinning polymer is a cationic polymer.

3. Composition according to claims 1 and 2 **characterised in that** it has a bulk density in the range of 0.5 to 0.65 g/ml measured at 20°C.

4. Composition according to any of the preceding claims **characterised in that** gas is selected from air, nitrogen and carbon dioxide.

5. Composition according to any of the preceding claims **characterised in that** gas dispersed in the composition has an average number diameter in the range of 100µ to 5 mm, preferably 250 µ to 3 mm.

6. Composition according to any of the preceding claims **characterised in that** conditioning agent is selected form oily substances, non-ionic substances, cationic amphiphilic ingredients, cationic polymers or their mixtures.

7. Composition according to any of the preceding claims **characterised in that** it is an emulsion.

8. Composition according claim 7 **characterised in that** it comprises one or more fatty alcohols.

9. Composition according to any of the preceding claims **characterised in that** it comprises at least one organic solvent.

10. Composition according to any of the preceding claims **characterised in that** it comprises at least one UV absorber.

11. Composition according to any of the preceding claims **characterised in that** it comprises synthetic mica coated with metal oxide or oxides and having a volume particle size distribution in the range of 1 to 750 µm at a concentration of 0.01 to 10% by weight, calculated to total composition.

12. Composition according to any of the preceding claims **characterised in that** it comprises at least one hair direct dye.

13. Composition according to any of the preceding claims **characterised in that** it has a pH in the range of 2 to 7.

14. Process for conditioning hair wherein a composition according to claims 1 to 13 is applied onto previously wetted or cleansed, using a cleansing composition, hair and after a processing time of 30 seconds to 30 min at a temperature range of 20 to 45°C, rinsed off from hair.

15. Ready to use product comprising composition according to claims 1 to 13 and a vessel having appropriate volume and a cap which enables storing the composition under atmospheric pressure.

16. Process for producing a conditioning composition for keratin fibres especially human hair according to claims 1 to 13 wherein composition produced without gas is mixed with an appropriate amount of gas to result in a bulk density in the range of 0.3 to 0.9 g/ml.

17. Use of composition according to any claims 1 to 13 for conditioning and/or colouring keratin fibres especially human hair.
